# EUROPEAN PATENT APPLICATION

(11) **EP 2 058 398 A1**
(43) Date of publication of application: **13.05.2009**
(21) Application number: 07120389.7
(22) Date of filing: 09.11.2007
(51) Int. Cl.: C12N 15/12, C07K 14/805

(54) **Production of recombinant human hemoglobin using pichia yeast**

(71) Applicant: Nipro Corporation, Osaka-shi, Osaka 531-8510 (JP)
(72) Inventor: Nakajou, Keisuke, Osaka 531-8510 (JP); Hoashi, Yohei, Osaka 531-8510 (JP); Kai, Toshiya, Osaka 531-8510 (JP); Uno, Tadayuki, Osaka 560-0083 (JP); Otagiri, Masaki, Kumamoto 861-8038 (JP)
(74) Representative: Helbing, Jörg

(57) **Abstract**

The present invention provides a production method of a functional recombinant human hemoglobin, which comprises transforming a yeast of the genus Pichia with an expression vector containing a DNA encoding a human hemoglobin α-chain (Hbα), which is under regulation of a promoter functional in a yeast of the genus Pichia, and an expression vector containing a DNA encoding a human hemoglobin β-chain (Hbβ), which is under regulation of a promoter functional in a yeast of the genus Pichia, culturing the yeast in a medium, and recovering the human hemoglobin from the obtained culture. The present invention also provides an expression vector containing AOX1 promoter, DNA encoding Hbβ, terminator functional in a yeast of the genus Pichia, AOX1 promoter, DNA encoding Hbα and terminator functional in the yeast of the genus Pichia in this order from the upstream side in transcriptional direction.

## Description

### TECHNICAL FIELD OF THE INVENTION

The present invention relates to production of a recombinant human hemoglobin and an expression vector therefor. More particularly, the present invention relates to a production method of a human hemoglobin using a yeast of the genus Pichia, as well as an expression vector comprising in tandem an expression cassette containing a DNA encoding a human hemoglobin β-chain and an expression cassette containing a DNA encoding a human hemoglobin α-chain.

### BACKGROUND OF THE INVENTION

Hemoglobin is a protein contained in red blood cells at a high concentration, and has a function to carry various gas molecules including oxygen. Structurally, it is a heterotetramer consisting of two α-chains and two β-chains.

As a preparation utilizing hemoglobin, a red blood cell substitute has been developed. There have so far been designed an intramolecular crosslinking type which is a hemoglobin modification form, a polymerization type, a polymer bond type, and hemoglobin endoplasmic reticulum in which hemoglobin is encapsulated in liposome. Particularly, there is a practical prospect of hemoglobin endoplasmic reticulum as a single administration oxygen transfusion for critical care, based on the property data of its shape, hemoglobin purity and the like, and the biological data of safety, in vivo retention, in vivo kinetics, oxygen-carrying ability and the like.

On the other hand, a preparation using a human blood-derived hemoglobin is associated with the risk of contamination with unknown virus and the like, and its use for human body is problematic in terms of safety. However, there is a possibility of provision of a safe hemoglobin preparation by a transformed microorganism using the gene recombination technique.

Until now, recombinant hemoglobin has been produced mainly using a system with Escherichia coli as a host cell. However, this method has problems in that (1) unless an enzyme (methionine aminopeptidase and the like) is coexpressed with hemoglobin and the like, addition of redundant first methionine to the N-terminal occurs, leading to a primary sequence different from that of a naturally occurring type, (2) aminolaevulinic acid needs to be added to a culture medium for the purpose of heme synthesis and the like.

Two cases have been reported so far as regards the production of recombinant hemoglobin using Saccharomyces cerevisiae (see Wagenbach, M. et al., Bio/Technol., 9: 57-61 (1991) and Coghlan, D. et al., Eur. J. Biochem., 207: 931-936 (1992)). In this system, since the first methionine is cleaved by an enzyme S. cerevisiae itself has, the primary sequence completely matches with that of a naturally occurring type, and addition of aminolaevulinic acid for heme synthesis is not necessary. However, this method is not entirely sufficient due to the problem of yield and the like, and therefore, an industrial production of recombinant human hemoglobin has not been practiced yet.

One of known industrial yeasts other than the genus Saccharomyces is a yeast of the genus Pichia. This yeast can grow with methanol as a sole carbon source, and when grown in methanol, an enzyme necessary for the treatment of methanol and its metabolism intermediate is disinhibited and expressed. Production of heterologous protein utilizing the methanol-utilizing pathway has been studied and applied to the production of albumin for blood preparations (see e.g., JP-A-6-22784). The expression amount thereof is known to be very high, and albumin of a 10 g order can be produced from 1 L of a medium.

However, there often exist cases where the expression efficiency of heterologous protein is low due to the combination (compatibility) of the object heterologous protein and a host cell. Particularly, in the case of a heterooligomer protein such as hemoglobin, since many conditions of balanced expression amounts of respective subunits, oligomer formation and the like need to be met, the desired high expression cannot be obtained easily. In fact, a yeast of the genus Pichia is used for industrial production of human protein in a very few cases.

An object of the present invention is to provide a means for producing a safe recombinant protein such as human hemoglobin or albumin, globulin and the like, which has sufficient function and activity similar to those of the naturally occurring type and is free of viral contamination and the like, easily in a large amount.

The present inventors have conducted intensive studies in an attempt to solve the above-mentioned problems and succeeded in recovering various proteins or peptides such as a human hemoglobin and the like, particularly a heterooligomer protein and the like, in a high yield by transforming Pichia pastoris, which is one of the yeasts of the genus Pichia, with an expression vector containing a DNA encoding plural proteins or peptide chains, for example, an expression vector containing a DNA encoding α-chain and β-chain of human hemoglobin, and cultivating the obtained transformant in a medium, which resulted in the completion of the present invention.

### SUMMARY OF THE INVENTION

Accordingly, the present invention provides
(1) a production method of a functional recombinant human hemoglobin, which comprises transforming a yeast of the genus Pichia with an expression vector containing a DNA encoding a human hemoglobin α-chain, which is under regulation of a promoter functional in a yeast of the genus Pichia, and an expression vector containing a DNA encoding a human hemoglobin β-chain, which is under regulation of a promoter functional in a yeast of the genus Pichia, culturing the yeast in a medium, and recovering the human hemoglobin from the obtained culture;
(2) the method of the above-mentioned (1), wherein the DNA encoding the human hemoglobin α-chain and the DNA encoding the human hemoglobin β-chain are on the same vector;
(3) the method of the above-mentioned (2), wherein the DNA encoding the human hemoglobin β-chain is at the upstream side in the transcriptional direction;
(4) the method of any of the above-mentioned (1) - (3), wherein the DNA encoding the human hemoglobin α-chain and the DNA encoding the human hemoglobin β-chain are under regulation of different promoters;
(5) the method of any of the above-mentioned (1) - (4), wherein the DNA encoding the human hemoglobin α-chain and the DNA encoding the human hemoglobin β-chain are under regulation of an alcoholoxidase 1 promoter;
(6) the method of the above-mentioned (5), wherein the transformed yeast of the genus Pichia is cultivated in a liquid medium requiring methanol as a single carbon source;
(7) the method of the above-mentioned (6), wherein the culture is a feeding culture;
(8) the method of any of the above-mentioned (1) - (7), wherein the yeast of the genus Pichia is Pichia pastoris; and
(9) an expression vector comprising an alcoholoxidase 1 promoter, a DNA encoding the human hemoglobin β-chain, a terminator functional in a yeast of the genus Pichia, an alcoholoxidase 1 promoter, a DNA encoding a human hemoglobin α-chain and a terminator functional in a yeast of the genus Pichia in this order from the upstream side in the transcriptional direction.
   The present invention also provides
(10) a method of producing an expression vector comprising a DNA encoding plural proteins, which is under control of a promoter functional in a yeast of the genus Pichia, wherein the vector and a DNA encoding one of the plural proteins are ligated without a dephosphorylation treatment of the vector;
(11) the method of the above-mentioned (10), wherein (i) an expression vector containing a DNA encoding one or more proteins, which is under control of a promoter functional in a yeast of the genus Pichia, wherein the expression vector is digested with a restriction enzyme capable of cleavage thereof other than the inside of the promoter and the DNA and the both ends produced thereby are not dephosphorylated is provided, (ii) a DNA encoding other protein, which is under control of a promoter functional in a yeast of the genus Pichia, wherein the both ends are complementary to the end of the expression vector of the above-mentioned (i) is provided, and (iii) the expression vector of the above-mentioned (i) and the DNA of the above-mentioned (ii) are ligated;
(12) a production method of a recombinant protein, which comprises culturing, in a medium, a yeast of the genus Pichia transformed with an expression vector obtained by the method of the above-mentioned (10) or (11), and recovering the plural proteins from the obtained culture;
(13) the method of the above-mentioned (12), wherein the plural proteins are polymerized in the culture to form a functional protein complex; and the like.

Since the human hemoglobin provided by the method of the present invention is produced using a yeast of the genus Pichia as a host by the gene recombination technique, it is free of a risk of contamination with an unknown virus and the like, which is a problem specific to blood-derived preparations, and can be used safely for human body and the like. Using a yeast of the genus Pichia, moreover, a human hemoglobin can be produced in a large amount, which has similar function and properties as the naturally occurring type. Accordingly, administration thereof to a human body etc. causes less influence such as side effects and the like.

### BRIEF DESCRIPTION OF THE DRAWINGS

Fig. 1 is an explanatory drawing of the principle of connection between hemoglobin α-chain expression cassette and β-chain expression cassette.
Fig. 2 is an explanatory drawing of the outline of the procedures for preparing hemoglobin in the Examples.
Fig. 3 is an explanatory drawing of the recombination method of hemoglobin with Pichia chromosome in the Examples.

### DETAILED DESCRIPTION OF THE INVENTION

As a DNA encoding the human hemoglobin α-chain to be used in the present invention (hereinafter sometimes to be abbreviated as "Hbα"), a DNA containing a base sequence encoding an amino acid sequence the same or substantially the same as the amino acid sequence shown in SEQ ID NO: 2, and as a DNA encoding a human hemoglobin β-chain (hereinafter sometimes to be abbreviated as "Hbβ"), a DNA containing a base sequence encoding an amino acid sequence the same or substantially the same as the amino acid sequence shown in SEQ ID NO: 4 can be mentioned.

As the amino acid sequence the same or substantially the same as the amino acid sequence shown in SEQ ID NO: 2 (or SEQ ID NO: 4), an amino acid sequence having a homology of not less than about 80%, preferably not less than about 90%, more preferably not less than about 95%, particularly preferably not less than about 97% with the amino acid sequence shown in SEQ ID NO: 2 (or SEQ ID NO: 4) and the like can be mentioned. As used herein, by the "homology" is meant the proportion (%) of the same amino acid and similar amino acid residues relative to the total overlapping amino acid residues in the optimal alignment when two amino acid sequences are aligned using a mathematical algorithm known in the art (preferably, the algorithm is capable of considering introduction of a gap into one or both of the sequences for the optimal alignment). The "similar amino acid" means an amino acid similar in the physicochemical properties. For example, amino acids classified in the same group such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids (Ser, Thr) having a hydroxyl group, amino acids (Gly, Ala, Ser, Thr, Met) with small side chain and the like can be mentioned. It is predicted that substitution with such similar amino acids will not alter protein phenotypes (namely, preservative amino acid substitution). Specific examples of preservative amino acid substitution are well known in the art and are described in various literatures (see e.g., Bowie et al., Science, 247: 1306-1310 (1990)).

The homology of the amino acid sequence in the present specification can be calculated using homology calculation algorithm NCBI BLAST (National Center for Biotechnology Information Basic Local Alignment Search Tool) under the following conditions (expectancy=10; allowing gap; matrix=BLOSUM62; filtering=OFF). Other algorithms for determining the homology of the amino acid sequence include, for example, the algorithm described in Karlin et al., Proc. Natl. Acad. Sci. USA, 90: 5873-5877 (1993) [this algorithm is incorporated in the NBLAST and XBLAST program (version 2.0) (Altschul et al., Nucleic Acids Res., 25: 3389-3402 (1997))], the algorithm described in Needleman et al., J. Mol. Biol., 48: 444-453 (1970) [this algorithm is incorporated in the GAP program in the GCG software package], the algorithm described in Myers and Miller, CABIOS, 4: 11-17 (1988) [this algorithm is incorporated in the ALIGN program (version 2.0) which is a part of the CGC sequence alignment software package], the algorithm described in Pearson et al., Proc. Natl. Acad. Sci. USA, 85: 2444-2448 (1988) [this algorithm is incorporated in the FASTA program in the GCG software package] and the like, and they can also be used preferably.

More preferably, an amino acid sequence substantially same as the amino acid sequence shown in SEQ ID NO: 2 (or SEQ ID NO: 4) has homology of not less than about 80%, preferably not less than about 90%, more preferably not less than about 95%, particularly preferably not less than about 97%, to the amino acid sequence shown in SEQ ID NO: 2 (or SEQ ID NO: 4).

A protein containing an amino acid sequence substantially same as the amino acid sequence shown in SEQ ID NO: 2 (or SEQ ID NO: 4) means a protein containing an amino acid sequence substantially same as the aforementioned amino acid sequence shown in SEQ ID NO: 2 (or SEQ ID NO: 4) and having a substantially equivalent activity to that of the protein containing the amino acid sequence shown in SEQ ID NO: 2 (or SEQ ID NO: 4).

The substantially equivalent activity includes, for example, the physiological activity of human hemoglobin, for example, an activity to carry a gas molecule (e.g., oxygen and the like), and the like. The "substantially equivalent" means that the activities are qualitatively same. Therefore, the activity of oxygen-carrying ability and the like is preferably equivalent (e.g., about 0.5- to about 2-fold). However, quantitative elements such as the level of the activity, molecular weight of the protein and the like may be different.

The activity of oxygen-carrying ability and the like (e.g., autoxidation rate constant, oxygen affinity, cooperativity and the like) can be measured according to, but not limited to, a method known per se, for example, the method described in JP-A-2004-500871 and the like.

In addition, Hbα (or Hbβ) to be used in the present invention includes, for example, a protein containing (1) an amino acid sequence wherein one or more (preferably about 1 - 30, more preferably about 1 - 10, particularly preferably 1 - several (2, 3, 4 or 5)) amino acids are deleted from the amino acid sequence shown in SEQ ID NO: 2 (or SEQ ID NO: 4), (2) an amino acid sequence wherein one or more (preferably about 1 - 30, more preferably about 1 - 10, particularly preferably 1 - several (2, 3, 4 or 5)) amino acids are added to the amino acid sequence shown in SEQ ID NO: 2 (or SEQ ID NO: 4), (3) an amino acid sequence wherein one or more (preferably about 1 - 30, more preferably about 1 - 10, particularly preferably 1 - several (2, 3, 4 or 5)) amino acids are inserted in the amino acid sequence shown in SEQ ID NO: 2 (or SEQ ID NO: 4), (4) an amino acid sequence wherein one or more (preferably about 1 - 30, more preferably about 1 - 10, particularly preferably 1 - several (2, 3, 4 or 5)) amino acids are substituted by other amino acids in the amino acid sequence shown in SEQ ID NO: 2 (or SEQ ID NO: 4), or (5) a combination of these, and the like.

When the amino acid sequence is inserted, deleted or substituted as mentioned above, the site of the insertion, deletion or substitution is not particularly limited as long as the activity of protein is maintained.

Human hemoglobin in the present invention is used to mean not only adult hemoglobin (HbA1) but encompass HbA2 wherein α-chain and δ-chain are bonded, fetal hemoglobin (HbF) wherein α-chain and γ-chain are bonded, and the like.

For the production of HbA2 and HbF, therefore, a DNA encoding δ-chain and γ-chain instead of β-chain is used. The amino acid sequence and base sequence of δ-chain are registered, for example, in GenBank accession Nos. NP_000510 and NM_000519. On the other hand, the amino acid sequence and base sequence of γ-chain are registered, for example, in GenBank accession Nos. NP_000550 and NM_000559.

Examples of the DNA encoding Hbα (or Hbβ) include human genomic DNA, cDNA derived from Hbα (and/or Hbβ)-producing cells (e.g., red blood cell and the like) of adult, synthetic DNA and the like. The genomic DNA and cDNA encoding Hbα (or Hbβ) can also be directly amplified by Polymerase Chain Reaction (hereinafter to be abbreviated as "PCR method") and Reverse Transcriptase-PCR (hereinafter to be abbreviated as "RT-PCR method"), using a genomic DNA fraction and total RNA or an mRNA fraction prepared from a producing-cell or tissue (e.g., blood and the like) thereof as respective templates. Alternatively, the genomic DNA and cDNA encoding Hbα (or Hbβ) can be respectively cloned by colony or plaque hybridization method, PCR method and the like, from a genomic DNA library and cDNA library prepared by inserting genomic DNA and total RNA or an mRNA fraction prepared from the above-mentioned cell or tissue into suitable vectors. The vector to be used for the library may be any such as bacteriophage, plasmid, cosmid, phagemid and the like.

Examples of the DNA encoding Hbα (or Hbβ) include DNA containing the base sequence shown in SEQ ID NO: 1 (or SEQ ID NO: 3), DNA having a base sequence hybridizing with the base sequence shown in SEQ ID NO: 1 (or SEQ ID NO: 3) under stringent conditions, and encoding a protein having substantially equivalent activity (e.g., oxygen-carrying ability and the like) to that of the aforementioned Hbα (or Hbβ) and the like.

As the DNA capable of hybridizing to the base sequence shown in SEQ ID NO: 1 (or SEQ ID NO: 3) under stringent conditions, for example, DNA containing a base sequence having homology of not less than about 70%, preferably not less than about 80%, more preferably not less than about 90%, particularly preferably not less than about 95%, to the base sequence shown in SEQ ID NO: 1 (or SEQ ID NO: 3) and the like are used.

The homology of the base sequence in the present specification can be calculated using a homology calculation algorithm NCBI BLAST (National Center for Biotechnology Information Basic Local Alignment Search Tool) under the following conditions (expectancy=10; allowing gap; filtering=ON; match score=1; mismatch score=-3). Preferable examples of other algorithms usable for determining homology of the base sequence include the above-mentioned homology calculation algorithms for amino acid sequence.

Hybridization can be performed according to a method known per se or a method according to the method, for example, the method described in Molecular Cloning, ver. 2 (J. Sambrook et al., Cold Spring Harbor Lab. Press, 1989) and the like. When a commercially available library is used, moreover, hybridization can be performed according to the method described in the attached instruction manual. Preferably, hybridization can be performed under stringent conditions.

The high stringent conditions include, for example, a hybridization reaction at 45°C in 6×SSC (sodium chloride/sodium citrate), and washing one or more times at 65°C in 0.2×SSC/0.1% SDS and the like.

Those of ordinary skill in the art can easily adjust to the desired stringency by appropriately changing the salt concentration of hybridization solution, temperature of hybridization reaction, probe concentration, length of probe, number of mismatches, hybridization reaction time, salt concentration of washing solution, temperature of washing and the like.

A DNA encoding Hbα (or Hbβ) can be cloned by amplifying by PCR method using a synthetic DNA primer having a part of the base sequence encoding Hbα (or Hbβ), or hybridizing DNA incorporated into a suitable expression vector to with a labeled DNA fragment or synthetic DNA encoding a part or full region of Hbα (or Hbβ).

A DNA encoding Hbα (or Hbβ) can be obtained from human genomic DNA or RNA (cDNA) as mentioned above. It is also possible to construct a DNA encoding the full-length Hbα (or Hbβ) by chemically synthesizing a DNA chain or connecting a synthesized, partly overlapping oligo DNA short chain by PCR method. The advantage of constructing a full-length DNA by a combination of chemical synthesis and PCR method is that the codon to be used can be designed over the full length of the gene in accordance with the host to be introduced into the gene. Plural codons encoding the same amino acid are not uniformly used and the frequency of use varies depending on the species. In general, a codon contained in a gene highly expressing in a certain species contains a large amount of codon frequently used in the species. On the other hand, in many of the genes with a low expression amount, the presence of a codon used less frequently often becomes a bottleneck in the high expression. There are many reports on the examples wherein, in the expression of a heterologous gene, the expression amount of the heterologous protein was increased by substituting the gene sequence with a codon highly frequently used in the host organism, and therefore, such alteration of the codon to be used is expected to be effective for increasing the expression amount of a heterologous gene.

For the above-mentioned reasons, a DNA encoding Hbα (or Hbβ) can be altered to a codon more suitable for a yeast of the genus Pichia into which the DNA is to be introduced (that is, a codon highly frequently used in the host organism). The frequency of use of the codon in each yeast of the genus Pichia is defined by the ratio of use of each codon to all the genes present on the genomic sequence of the host, for example, by the number of use per 1000 codons. In addition, the frequency of use of codon can also be approximately calculated from plural representative gene sequences in the case of a biological entity for which the complete genomic sequences have not been identified. As for the data of frequent use of codon in yeast of the genus Pichia to be genetically modified, for example, the genetic code usage frequency database (http://www.kazusa.or.jp/codon/index.html) disclosed in the homepage of Kazusa DNA Research Institute can be used. Alternatively, a document indicating the frequency of use of codon in each yeast of the genus Pichia may be referred to, or the data of frequency of use of codon in the yeast of the genus Pichia to be used may be constructed by oneself. By referring to the obtained data and the gene sequence to be introduced, the codon used in the gene sequence, which shows a low frequency of use in the host yeast, can be substituted to a codon encoding the same amino acid and showing a high frequency of use.

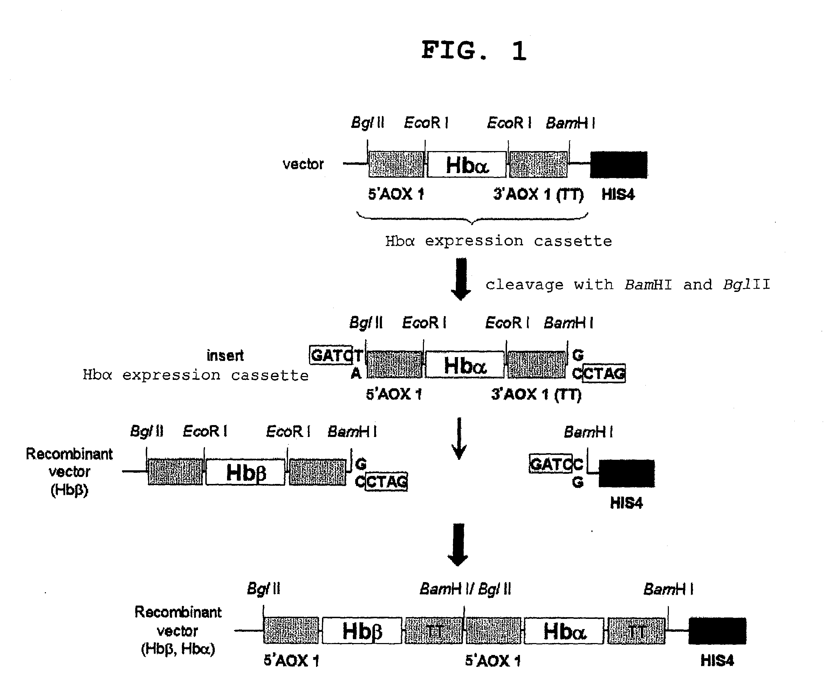

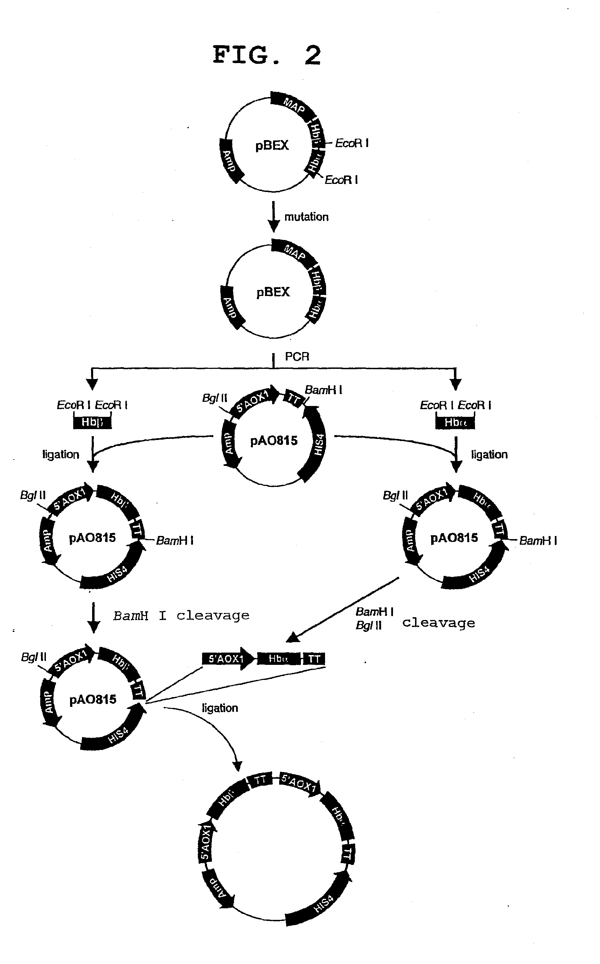

Another advantage of constructing a DNA encoding the full-length Hbα (or Hbβ) by chemically synthesizing a DNA chain, or connecting a synthesized, partly overlapping oligo DNA short chain by PCR method is easy construction of an expression vector thereafter. For example, in the below-mentioned Examples, an expression cassette of Hbβ and an expression cassette of Hbα are connected in tandem, utilizing the complementarity of sticky end produced by digestion with restriction enzymes BamH I and Bgl II (see Fig. 1. Utilizing the complementarity of sticky end, multicopy expression cassette can be repeatedly integrated in tandem into a vector). Therefore, the DNA encoding Hbα and the DNA encoding Hbβ are preferably free of a BamH I recognition site. For example, Hbβ cDNA registered as GenBank accession No. NM_000518 in the NCBI database has a BamH I recognition site (GGATCC) at the 347th - 352nd positions. In the base sequence shown in SEQ ID NO: 3, the base sequence of the corresponding part (base Nos. 297 - 302) is TGACCC, where a mutation has been introduced to not contain a BamH I recognition site. In the below-mentioned Examples, moreover, an EcoR I recognition sequence is added to both ends of a DNA encoding Hbα and a DNA encoding Hbβ to construct an expression cassette by inserting a DNA encoding Hbα and a DNA encoding Hbβ between a promoter and a terminator functional in a yeast of the genus Pichia (see Fig. 2). Therefore, a DNA encoding Hbα and a DNA encoding Hbβ is preferably free of a EcoR I recognition site. For example, Hbβ cDNA registered as GenBank accession No. NM_000518 in the NCBI database has an EcoR I recognition site (GAATTC) at the 414th to 419th positions. In the base sequence shown in SEQ ID NO: 3, the base sequence of the corresponding part (base Nos. 370 - 375) is GAGTTT, where a mutation has been introduced to not contain an EcoR I recognition site. When a restriction enzyme recognition site in a protein-coding sequence is to be altered, it is desirable to substitute a base, thereby to not change the amino acid sequence of Hbα (or Hbβ) utilizing degeneration of the codon.

A DNA cloned as mentioned above can be used as it is depending on the object or used after digestion with a restriction enzyme or adding a linker. The DNA may have ATG as a translation initiation codon at the 5' end side thereof and TAA, TGA or TAG as a translation stop codon at the 3' end side thereof. These translation initiation codon and translation stop codon can be added using a suitable synthetic DNA adapter.

An expression vector containing a DNA encoding Hbα (or Hbβ) can be produced, for example, by ligating a DNA fragment encoding Hbα (or Hbβ) cloned as mentioned above to a downstream of a promoter in a suitable expression vector using a restriction enzyme and a DNA ligase.

The vector to be used is not particularly limited as long as it can be maintained stably by autonomous replication in a fungus body of yeast of the genus Pichia or integration into a yeast genome. Examples of the autonomously-replicable vector include YEp vector, YRp vector, YCp vector and the like. In addition, examples of the vector to be integrated into a yeast genome include YIp vector and YRp vector.

Examples of the promoter functional in the yeast of the genus Pichia include promoters derived from a yeast, such as PHO5 promoter, PGK promoter, GAP promoter, ADH promoter derived from S. cerevisiae and the like, alcohol oxidase (AOX) 1 promoter, AOX2 promoter, dihydroxyacetone synthase promoter, P40 promoter, ADH promoter, folic acid dehydrogenase promoter derived from P. pastoris and the like. In addition, the above-mentioned promoter derived from a yeast may be a mutant promoter modified to further improve the gene expression efficiency, for example, mutant AOX2 (mAOX2) promoter [Ohi et al., Mol. Gen. Genet., 243, 489-499 (1994); JP-A-4-299984] and the like. Preferably, the promoter is a promoter of an enzyme gene necessary for treating methanol or a metabolic intermediate thereof, in order to use a methanol-metabolizing system in the yeast of the genus Pichia, such as AOX1 promoter, mAOX2 promoter and the like, more preferably AOX1 promoter.

The expression vector containing the DNA encoding Hbα (or Hbβ) of the present invention preferably further contains transcription terminator sequence (terminator) functional in a yeast of the genus Pichia (e.g., AOX1 terminator etc.), enhancer sequence, selection marker gene usable for selecting yeast (auxotrophic gene, for example, HIS4, LEU2, ARG4 and URA3 gene derived from P. pastoris or S. cerevisiae, and the like, or antibiotic resistance gene, for example, resistance gene to cycloheximide, G-418, chloramphenicol, bleomycin, hygromycin etc., and the like) and the like, and when desired, may contain replicable unit functional in yeast. For preparation of the vector in a large amount, moreover, the vector more preferably contains a replicable unit functional in Escherichia coli and a selection marker gene usable for selecting Escherichia coli (e.g., resistance gene to ampicillin and tetracycline etc.).

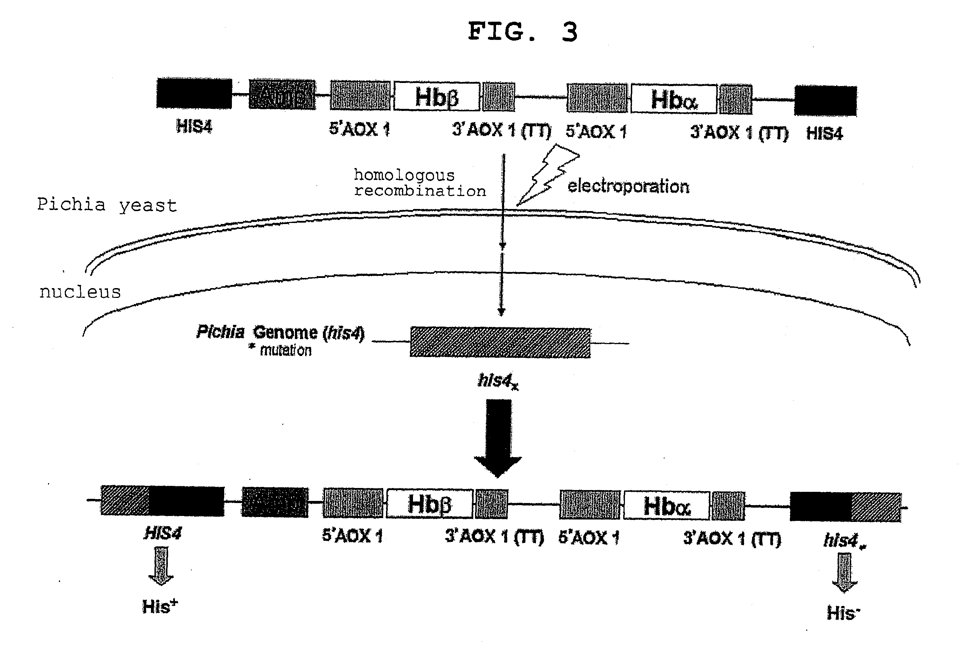

When the expression vector is of a type incorporated into a yeast genome, the vector preferably further contains a sequence homologous to a yeast genome necessary for homologous recombination. As such homology sequence, the aforementioned auxotrophic gene sequence can be mentioned. Accordingly, in one preferable embodiment, the expression vector of the present invention is one wherein an expression cassette of the above-mentioned Hbα (or Hbβ) is inserted in an auxotrophic gene (in the present specification, the "expression cassette" means a unit enabling gene expression, whose minimal unit is a protein-coding sequence configured under regulation of a promoter, with preference given to a unit comprising promoter-protein-coding region-terminator) (see Fig. 3).

An expression cassette of Hbα and an expression cassette of Hbβ may be carried on individual vectors or the same vector. In the former case, two vectors desirably contain different selection marker genes. However, in a preferable embodiment of the present invention, the expression cassette of Hbα and the expression cassette of Hbβ are configured on the same vector. In this case, the expression cassette of Hbα and the expression cassette of Hbβ may be configured in any order. For example, the expression cassettes may be configured head-to-head or tail-to-tail on an opposite chain. Preferably, they are configured in the same transcriptional direction, more preferably, the expression cassettes are configured in the order of the expression cassette of Hbβ and the expression cassette of Hbα, from the upstream side in the transcriptional direction. When the DNA encoding Hbα and the DNA encoding Hbβ are transcribed in the same direction, the transcription may be performed monocistronically from the same promoter or dicistronically from separate promoters. In the case of monocistronic transcription, the expression vector does not contain a terminator at the 3' side of the coding sequence in the upstream. In addition, an internal ribosome entry site (IRES) is preferably contained at the 5' side of the coding sequence in the downstream. However, in a preferable embodiment of the present invention, the DNA encoding Hbα and the DNA encoding Hbβ are transcribed dicistronically from separate promoters.

In a particularly preferable embodiment of the present invention, an expression vector containing AOX1 promoter, DNA encoding Hbβ, terminator functional in a yeast of the genus Pichia (preferably AOX1 terminator), AOX1 promoter, DNA encoding Hbα and terminator functional in the yeast of the genus Pichia (preferably AOX1 terminator) in this order from the upstream side in transcriptional direction is used.

For construction of an expression vector comprising the above-mentioned expression cassette of Hbβ and the expression cassette of Hbα ligated in tandem, generally, a site immediately upstream (when expression cassette of Hbα is contained) of the promoter of the expression vector containing one of the expression cassettes or a site immediately downstream (when expression cassette of Hbβ is contained) of the terminator thereof is cleaved with a suitable restriction enzyme (e.g., in Fig. 2, Hbβ-pAO815 is cleaved with BamH I) to linearize the vector, and ligated using the other expression cassette cleaved by treating with a restriction enzyme providing the same sticky end (or blunt end) (e.g., in Fig. 2, expression cassette of Hbα is cleaved out from Hbα-pAO815 by double digestion with BamH I and Bgl II) and a DNA ligase. At this time, in general, a dephosphorylation treatment of the end (e.g., calf intestine alkaline phosphatase (CIAP) treatment) is performed to prevent self-ligation on the vector side. In some cases, the total ligation efficiency can be enhanced when the dephosphorylation treatment is not applied.

The expression vector obtained as mentioned above can be introduced into the fungus body of the target yeast of the genus Pichia using, for example, a known transformation technique such as competent cell method, protoplast method, calcium phosphate coprecipitation method, polyethylene glycol method, lithium method, electroporation method, microinjection method, liposome fusion method, particle gun method and the like.

While the yeast of the genus Pichia to be used in the present invention is not particularly limited, for example, P. pastoris, Pichia acaciae, Pichia angusta, Pichia anomala, Pichia capsulata, Pichia ciferrii, Pichia etchellsii, Pichia fabianii, Pichia farinosa, Pichia guilliermondii, Pichia inositovora, Pichia jadinii, Pichia methanolica, Pichia norvegensis, Pichia ofunaensis, Pichia pinus and the like can be used. Preferred is P. pastoris, particularly, auxotrophic mutant P. pastoris strain (e.g., P. pastoris GTS115 strain (HIS4⁻) [NNRL Y-15851], P. pastoris GS190 strain (ARG4⁻) [NNRLY-18014), P. pastoris PPF1 (HIS4^{-,} URA4⁻) [NNRL Y-18017] and the like).

By cultivating the transformed yeast of the genus Pichia by a method generally used in the art, a functional human hemoglobin can be produced. The medium to be used needs to contain at least a carbon source and an inorganic or organic nitrogen source necessary for the growth of the host cell. Examples of the carbon source include methanol, glycerol, glucose, sucrose, dextran, soluble starch and the like. In addition, examples of the inorganic or organic nitrogen source include ammonium salts, nitrate salts, amino acid, corn steep liquor, peptone, casein, meat extract, yeast extract, soybean cake, potato extract and the like. When desired, moreover, other nutrients, for example, inorganic salts such as calcium chloride, sodium dihydrogenphosphate, magnesium chloride and the like, vitamins such as biotin and the like, antibiotic and the like can be added.

Examples of the medium to be used include conventional natural medium (e.g., YPD medium, YPM medium, YPG medium etc.) and synthetic medium. As the pH and culture temperature of the medium, those suitable for the growth of yeast and production of human hemoglobin are employed. For example, pH of about 5 - about 8, culture temperature of about 20°C - about 30°C are preferable. In addition, aeration and agitation are performed as necessary. The culture is generally performed for about 48 - about 120 hr.

For example, when a promoter whose expression is induced by methanol, such as AOX1 promoter, mAOX2 promoter and the like, is used as a promoter functional in the fungus body of a yeast of the genus Pichia, a method of liquid aeration-agitation culture using natural medium controlled to pH about 6.0, which contains glycerol as a carbon source for the growth of fungus body and methanol as an Hbα and Hbβ expression inducer is most preferable. When the expression of Hbα and Hbβ is not preferable for the growth of fungus body, a method including first increasing the amount of fungus body with a carbon source other than methanol, and inducing the expression of Hbα and Hbβ by addition of methanol is more preferable. In a culture in a jarfermenter, moreover, a high density culture method is suitable for the production of human hemoglobin. The culture may be performed by any of batch culture, feeding culture and continuous culture, with preference given to feeding culture method. That is, for a certain period, a method including culturing the host fungus body in a medium (initial medium) containing a carbon energy source suitable for the growth (e.g., glucose etc.) and/or a nutrient source, and confining human hemoglobin in the system until completion of the culture while additionally supplying a substrate controlling the expression of Hbα and Hbβ (that is, methanol) to the medium from a certain point in time according to the situation can be used (see e.g., JP-A-3-83595).

Human hemoglobin produced in the culture can be collected in the yeast fungus body by applying the culture after completion of cultivation to centrifugation and/or filtration, and subsequently isolated and purified from the fungus body according to a method known per se. As such method, a method utilizing the solubility such as salting out, solvent precipitation and the like; a method mainly utilizing difference in the molecular weight such as dialysis, ultrafiltration, gel filtration method, SDS-polyacrylamide gel electrophoresis and the like; a method utilizing difference in the electric charge such as ion exchange chromatography and the like; a method utilizing specific affinity such as affinity chromatography and the like; a method utilizing difference in hydrophobicity such as reversed-phase high performance liquid chromatography and the like; a method utilizing difference in the isoelectric point such as isoelectric focusing and the like; and the like can be used. These methods can be appropriately combined.

Examples of a method for confirming the isolated and purified human hemoglobin include known Western blotting method, activity measurement method and the like. In addition, the structure of the purified hemoglobin can be clarified by amino acid analysis, N-terminal amino acid sequence, primary structure analysis and the like. Human hemoglobin obtained in this way has the same primary structure (that is, removal by cleavage of first methionine) and higher-order structure as those of the naturally occurring type, and shows the physiological activity of the same quality as the naturally occurring type.

The recombinant human hemoglobin produced in the present invention can be administered to the living body, for example, as a red blood cell substitute. For administration as a red blood cell substitute, hemoglobin can be administered by encapsulating in liposome or dispersing in emulsion. The methods of preparation making and method of administration to human are described in, for example, JP-A-2004-307404, JP-A-2006-104069, JP-A-2002-161047, JP-A-2001-348341, JP-A-08-003063, JP-A-08-003062, JP-A-07-017874, JP-A-06-321802, JP-A-06-072892 and the like.

### SEQUENCE LISTING FREE TEXT

SEQ ID NO: 5
   primer.
SEQ ID NO: 6
   primer.
SEQ ID NO: 7
   primer.
SEQ ID NO: 8
   primer.
SEQ ID NO: 9
   primer.
SEQ ID NO: 10
   primer.
SEQ ID NO: 11
   primer.
SEQ ID NO: 12
   primer.
SEQ ID NO: 13
   Hbα cDNA having EcoR I site at 5' and 3'-ends.
SEQ ID NO: 14
   Hbβα cDNA having EcoR I site at 5' and 3'-ends.
SEQ ID NO: 15
   primer.
SEQ ID NO: 16
   primer.
SEQ ID NO: 17
   primer.
SEQ ID NO: 18
   primer.
SEQ ID NO: 19
   primer.

The present invention is explained in more detail in the following by referring to Examples, which are mere exemplifications and do not limit the scope of the present invention.

### EXAMPLES

### (1) amplification of hemoglobin gene

Using plasmid pBEX transfected with human hemoglobin gene (hereinafter referred to as "pBEX-Hb". Available from Tadayuki Uno (Graduate School and School of Pharmaceutical Sciences, Osaka University), a coinventor of the present application) as a template, and Hbα-sense primer of SEQ ID NO: 5 and Hbα-antisense primer of SEQ ID NO: 6 as synthetic primers of the DNA sequence of hemoglobin α-chain, and Hbβ-sense primer of SEQ ID NO: 7 and Hbβ-antisense primer of SEQ ID NO: 8 as synthetic primers of the DNA sequence of hemoglobin β-chain, respectively, mutation of EcoR I restriction enzyme site, which would become a obstruction of subsequent manipulation, was performed (QuikChange XL Site-Directed Mutagenesis Kit, Stratagene). As for mutation reaction conditions, DNA was treated for 30 sec at 95°C, after which a 12-cycle reaction of denaturation (95°C, 30 sec), annealing (55°C, 1 min) and extension (68°C, 10 min) was performed. After the reaction, the template plasmid was digested by Dpn I, and each of obtained pBEX-Hb(αΔ) and pBEX-Hb(βΔ) were transfected into XL-10-Gold ultracompetent cells to perform transformation. The transformants, which were transfected with the objective plasmids pBEX-Hb(αΔ) or pBEX-Hb(βΔ), were screened in ampicillin-added medium, and the plasmids were purified from the obtained transformants (QIAprep Spin Miniprep Kit, manufactured by QIAGEN). The plasmids were digested with EcoR I (manufactured by Takara Shuzo Co., Ltd.), and the mutation was confirmed.

Using the obtained pBEX-Hb(αΔ) and pBEX-Hb(βΔ) as templates, and Hbα-sense primer of SEQ ID NO: 9 and Hbα-antisense primer of SEQ ID NO: 10 as synthetic primers of the DNA sequence of hemoglobin α-chain, and Hbβ-sense primer of SEQ ID NO: 11 and Hbβ-antisense primer of SEQ ID NO: 12 as synthetic primers of the DNA sequence of hemoglobin β-chain, PCR was performed using a polymerase (KOD-plus-, manufactured by Toyobo Co., Ltd.). As for PCR reaction conditions, DNA was treated for 2 min at 94°C, after which a 30-cycle reaction of denaturation (94°C, 15 sec), annealing (65°C, 30 sec) and extension (68°C, 1 min) was performed. By this PCR, DNA fragments, wherein EcoR I restriction enzyme recognition sites were added to 5' end and 3' end of the DNA sequences encoding hemoglobin α-chain and β-chain, respectively, were obtained. The DNA fragments amplified by PCR were purified by phenol extraction, ethanol precipitation, then digested with restriction enzyme EcoR I (manufactured by Takara Shuzo Co., Ltd.). After the restriction enzyme treatment, DNA fragments were subjected to 2% agarose electrophoresis, and bands corresponding to each of DNA fragments (DNA fragment encoding hemoglobin α-chain;Hbα, SEQ ID NO: 13 and DNA fragment encoding hemoglobin β-chain; Hbβ, SEQ ID NO: 14) were cut out, and subjected to gel extraction (QIAquick Gel Extraction Kit, manufactured by QIAGEN).

### (2) ligation of hemoglobin gene

E. coli JM109 (manufactured by Takara Shuzo Co., Ltd.) was transfected with pA0815 (manufactured by Invitrogen), a shuttle vector for Escherichia coli and yeast, to perform transformation. The transformant, which was transfected with pA0815, was screened in ampicillin-added medium, and the plasmid was purified from the obtained transformant (QIAprep Spin Miniprep Kit, manufactured by QIAGEN). The plasmid was digested with EcoR I (manufactured by Takara Shuzo Co., Ltd.), double-digested with EcoR I and Sal I (manufactured by Takara Shuzo Co., Ltd.) and double-digested with BamH I and Bgl II (manufactured by Takara Shuzo Co., Ltd.), by which restriction enzyme map was constructed, and the plasmid was confirmed to be an objective plasmid vector.

Escherichia coli, wherein the transfection of pA0815 was confirmed, was further cultured, and the plasmid was purified from grown bacterial bodies (QIAGEN plasmid Maxi Kit, manufactured by QIAGEN). Purified pA0815 was digested with EcoR I (manufactured by Takara Shuzo Co., Ltd.), purified by phenol extraction and ethanol precipitation, after which dephosphorylation treatment was performed (Alkaline Phosphatase E. coli C75, manufactured by Takara Shuzo Co., Ltd.).

Each of Hbα and Hbβ was ligated to dephosphorylation-treated pA0815 (DNA Ligation kit Ver. 1, manufactured by Takara Shuzo Co., Ltd.) to give Hbα-pAO815 and Hbβ-pAO815, respectively. Each of the obtained Hbα-pAO815 and Hbβ-pAO815 was transfected into E. coli JM109 (manufactured by Takara Shuzo Co., Ltd.) to perform transformation. The transformants, which were transfected with either the objective plasmids Hbα-pA0815 or Hbβ-pAO815, were screened in ampicillin-added medium, and the plasmids were purified from the obtained transformants (QIAprep Spin Miniprep Kit, QIAGEN manufactured by). The plasmids were digested with EcoR I (manufactured by Takara Shuzo Co., Ltd.), and double-digested with BamH I and Bgl II (manufactured by Takara Shuzo Co., Ltd.), by which restriction enzyme map was constructed, and the plasmids were confirmed to be Hbα-pAO815 and Hbβ-pAO815.

Furthermore, full sequences of hemoglobin α-chain and β-chain gene were confirmed by ABI Prism 310 Genetic Analyzer (Applied Biosystems) using 5' AOX1 sequencing primer of SEQ ID NO: 15 (manufactured by Invitrogen), Hbα-sequence primer 1 of SEQ ID NO: 16 and Hbα-sequence primer 2 of SEQ ID NO: 17 for α-chain, and 5' AOX1 sequencing primer of SEQ ID NO: 15 (manufactured by Invitrogen), Hbβ-sequence primer 1 of SEQ ID NO: 18 and Hbβ-sequence primer 2 of SEQ ID NO: 19 for β-chain.

Escherichia coli, wherein the transfection of Hbα-pAO815 or Hbβ-pAO815 were confirmed, was further cultured, and the plasmids were purified from grown bacterial bodies (QIAGEN plasmid Maxi Kit, manufactured by QIAGEN). Purified Hbα-pAO815 was digested with BamH I and Bgl II (manufactured by Takara Shuzo Co., Ltd.), after which a band corresponding to Hbα expression cassette was cut out, and gel extraction was performed (QIAquick Gel Extraction Kit, manufactured by QIAGEN).

On the other hand, purified Hbβ-pAO815 was digested with BamH I (manufactured by Takara Shuzo Co., Ltd.) without performing dephosphorylation treatment, and purified by phenol extraction and ethanol precipitation.

Hbα expression cassette was ligated to Hbβ-pAO815 (DNA Ligation kit Ver. 1, manufactured by Takara Shuzo Co., Ltd.), which was enzyme-digested by BamH I, to give Hbβ-Hbα-pAO815. Obtained Hbβ-Hbα-pAO815 was transfected into E. coli DH5α (manufactured by Toyobo Co., Ltd.) to perform transformation. The transformant, which was transfected with the objective plasmid Hbβ-Hbα-pAO815, was screened in ampicillin-added medium, and the plasmid was purified from the obtained transformant (QIAprep Spin Miniprep Kit, manufactured by QIAGEN). The plasmid was digested with EcoR I (manufactured by Takara Shuzo Co., Ltd.), and double-digested with BamH I and Bgl II (manufactured by Takara Shuzo Co., Ltd.), by which restriction enzyme map was constructed, and the plasmid was confirmed to be Hbβ-Hbα-pAO815. Fig. 2 shows the procedure for construction of Hbβ-Hbα-pAO815.

### (3) expression of hemoglobin

Hbβ-Hbα-pAO815 was digested with restriction enzyme Sal I, purified by phenol extraction and ethanol precipitation, and subsequently transformed into HIS4 gene locus of Pichia yeast (GS115 strain) by homologous recombination using an electroporation apparatus (Gene Pulser II Electroporation System, manufactured by BIO-RAD) (see Fig. 3). The obtained transformant was cultured in BMMY liquid medium, and stocked in glycerol after confirmation of expression of hemoglobin.

### (4) purification of hemoglobin

The transformed Pichia yeast was cultured in BMGY liquid medium for 48 hr, and subsequently in BMMY medium for 96 hr as adding 1% methanol every 12 hr. The yeast was precipitated by centrifugation (6,000 g x 10 min), resuspended in disrupt buffer, and glass beads (425 - 600 µm) was added, after which the suspension was stirred intensively to disrupt the yeast.

The disrupted fungus body was separated by centrifugation at 10,000 g for 30 min, and the supernatant was collected. This was dialyzed against 10 mM Tris-HCl buffer (pH 7.5), and passed through Q-Sepharose^{™} Fast Flow column (manufactured by Amersham Biosciences), furthermore, bonded to the Q-Sepharose^{™} Fast Flow column by pH adjustment to 8.5, and hemoglobin was eluted by concentration gradient of 0 to 300 mM NaCl. This was replaced with 200 mM sodium acetate buffer (pH 5.5), bonded to Blue Sepharose CL-6B column (manufactured by Amersham Biosciences), and hemoglobin was eluted by concentration gradient of 0 to 3 M NaCl. Subsequently, this eluate was dialyzed against 0.5 M ammonium sulfate/100 mM sodium phosphate buffer (pH 7.0), bonded to HiTrap Butyl FF column (manufactured by Amersham Biosciences), and hemoglobin was eluted with 70% ethanol. Finally, CO was blown into the eluate and the eluate was preserved at 4°C.

### (5) confirmation of hemoglobin

The final sample was subjected to SDS polyacrylamide gel electrophoresis, and a single band of predicted molecular weight, about 15,000, was detected. Also, N-terminal amino acid analysis was performed, and it showed that the band completely corresponded with the amino acid sequence of human blood-derived hemoglobin. Furthermore, molecular weights of α-chain and β-chain were estimated by MALDI TOF-MS, 15,122 and 15,866, respectively, corresponding with calculated weights.

The production method of the present invention is free of a risk of contamination with virus and the like. Therefore, it is extremely useful in that it can conveniently provide human hemoglobin in large amounts, which can be safely administered to the living body for medical purposes.

While the present invention has been described with emphasis on preferred embodiments, it is obvious to those skilled in the art that the preferred embodiments can be modified. The present invention intends that the present invention can be embodied by methods other than those described in detail in the present specification. Accordingly, the present invention encompasses all modifications encompassed in the gist and scope of the appended "CLAIMS."

In addition, the contents disclosed in any publication cited herein, including patents and patent applications, are hereby incorporated in their entireties by reference, to the extent that they have been disclosed herein.

## Claims

1. A production method of a functional recombinant human hemoglobin, which comprises transforming a yeast of the genus Pichia with an expression vector containing a DNA encoding a human hemoglobin α-chain, which is under regulation of a promoter functional in a yeast of the genus Pichia, and an expression vector containing a DNA encoding a human hemoglobin β-chain, which is under regulation of a promoter functional in a yeast of the genus Pichia, culturing the yeast in a medium, and recovering the human hemoglobin from the obtained culture.

2. The method of claim 1, wherein the DNA encoding the human hemoglobin α-chain and the DNA encoding the human hemoglobin β-chain are on the same vector.

3. The method of claim 2, wherein the DNA encoding the human hemoglobin β-chain is at the upstream side in the transcriptional direction.

4. The method of any of claims 1 to 3, wherein the DNA encoding the human hemoglobin α-chain and the DNA encoding the human hemoglobin β-chain are under regulation of different promoters.

5. The method of any of claims 1 to 4, wherein the DNA encoding the human hemoglobin α-chain and the DNA encoding the human hemoglobin β-chain are under regulation of an alcoholoxidase 1 promoter.

6. The method of claim 5, wherein the transformed yeast of the genus Pichia is cultivated in a liquid medium requiring methanol as a single carbon source.

7. The method of claim 6, wherein the culture is a feeding culture.

8. The method of any of claims 1 to 7, wherein the yeast of the genus Pichia is Pichia pastoris.

9. An expression vector comprising an alcoholoxidase 1 promoter, a DNA encoding the human hemoglobin β-chain, a terminator functional in a yeast of the genus Pichia, an alcoholoxidase 1 promoter, a DNA encoding a human hemoglobin α-chain and a terminator functional in a yeast of the genus Pichia in this order from the upstream side in the transcriptional direction.


SEQUENCE LISTING

<110>  Nipro Corporation

<120>  Production of Recombinant Human Hemoglobin Using Pichia Yeast

<130>  072843ep/jh

<160>  19

<170>  PatentIn version 3.3

<210>  1
<211>  426
<212>  DNA
<213>  Homo sapiens


<220>
<221>  CDS
<222>  (1)..(426)

<400>  1
atg gtt ctg tct ccg gct gac aaa act aac gtt aaa gct gct tgg ggt       48
aaa gtt ggt gct cat gct ggt gaa tac ggt gct gaa gct ctg gaa cgt       96
atg ttc ctg tct ttc ccg act act aaa act tac ttc ccg cat ttc gac      144
ctg tct cat ggt tct gct cag gtt aaa ggt cat ggt aaa aaa gtt gct      192
gac gct ctg act aac gct gtt gct cat gtt gac gac atg ccg aac gct      240
ctg tct gct ctg tct gac ctg cat gct cat aaa ctg cgt gtt gac ccg      288
gtt aac ttc aaa ctg ctg tct cat tgc ctg ctg gtt act ctg gct gct      336
cat ctg ccg gct gag ttt act ccg gct gtt cat gct tct ctg gac aaa      384
ttc ctg gct tct gtt tct act gtt ctg act tct aaa tac cgt              426


<210>  2
<211>  142
<212>  PRT

<213>  Homo sapiens

<400>  2


<210>  3
<211>  441
<212>  DNA
<213>  Homo sapiens


<220>
<221>  CDS
<222>  (1)..(441)

<400>  3
atg gtt cat ctg act ccg gaa gaa aaa tct gct gtt act gct ctg tgg       48
ggt aaa gtt aac gtt gac gaa gtt ggt ggt gaa gct ctg ggt cgt ctg       96
ctg gtt gtt tac ccg tgg act cag cgt ttc ttc gaa tct ttc ggt gac      144
ctg tct act ccg gac gct gtt atg ggt aac ccg aaa gtt aaa gct cat      192
ggt aaa aaa gtt ctg ggt gct ttc tct gac ggt ctg gct cat ctg gac      240
aac ctg aaa ggt act ttc gct act ctg tct gaa ctg cat tgc gac aaa      288
ctg cat gtt gac ccg gaa aac ttc cgt ctg ctg ggt aac gtt ctg gtt      336
tgc gtt ctg gct cat cat ttc ggt aaa gag ttt act ccg ccg gtt cag      384
gct gct tac cag aaa gtt gtt gct ggt gtt gct aac gct ctg gct cat      432
aaa tac cat                                                          441


<210>  4
<211>  147
<212>  PRT
<213>  Homo sapiens

<400>  4

<210>  5
<211>  30
<212>  DNA
<213>  Artificial

<220>
<223>  Primer

<400>  5
gccggctgag tttactccgg ctgttcatgc                                        30

<210>  6
<211>  30
<212>  DNA
<213>  Artificial

<220>
<223>  Primer

<400>  6
gcatgaacag ccggagtaaa ctcagccggc                                        30

<210>  7
<211>  30
<212>  DNA
<213>  Artificial

<220>
<223>  Primer

<400>  7
cggtaaagag tttactccgc cggttcaggc                                        30

<210>  8
<211>  30
<212>  DNA
<213>  Artificial

<220>
<223>  Primer

<400>  8
gcctgaaccg gcggagtaaa ctctttaccg                                        30

<210>  9
<211>  34

<212>  DNA
<213>  Artificial

<220>
<223>  Primer

<400>  9
gcttaaaagg agatgaattc atggttctgt ctcc                                34


<210>  10
<211>  34
<212>  DNA
<213>  Artificial

<220>
<223>  Primer

<400>  10
cgggctttgt tagcagccga attcctatta acgg                                34


<210>  11
<211>  34
<212>  DNA
<213>  Artificial

<220>
<223>  Primer

<400>  11
ggagatgaat tcatggttca tctgactccg gaag                                34


<210>  12
<211>  40
<212>  DNA
<213>  Artificial

<220>
<223>  Primer

<400>  12
ccttttgaat tcctattaat ggtatttatg agccagagcg                          40


<210>  13
<211>  444
<212>  DNA
<213>  Artificial

<220>
<223>  Hb-alpha cDNA having EcoR I sites at 5' and 3' ends

<400>  13
gaattcatgg ttctgtctcc ggctgacaaa actaacgtta aagctgcttg gggtaaagtt      60

ggtgctcatg ctggtgaata cggtgctgaa gctctggaac gtatgttcct gtctttcccg     120

actactaaaa cttacttccc gcatttcgac ctgtctcatg gttctgctca ggttaaaggt     180

catggtaaaa aagttgctga cgctctgact aacgctgttg ctcatgttga cgacatgccg     240

aacgctctgt ctgctctgtc tgacctgcat gctcataaac tgcgtgttga cccggttaac 300

ttcaaactgc tgtctcattg cctgctggtt actctggctg ctcatctgcc ggctgagttt 360

actccggctg ttcatgcttc tctggacaaa ttcctggctt ctgtttctac tgttctgact 420

tctaaatacc gttaatagga attc 444

<210> 14
<211> 459
<212> DNA
<213> Artificial

<220>
<223> Hb-beta cDNA having EcoR I sites at 5' and 3' ends

<400> 14
gaattcatgg ttcatctgac tccggaagaa aaatctgctg ttactgctct gtggggtaaa 60

gttaacgttg acgaagttgg tggtgaagct ctgggtcgtc tgctggttgt ttacccgtgg 120

actcagcgtt tcttcgaatc tttcggtgac ctgtctactc cggacgctgt tatgggtaac 180

ccgaaagtta aagctcatgg taaaaaagtt ctgggtgctt tctctgacgg tctggctcat 240

ctggacaacc tgaaaggtac tttcgctact ctgtctgaac tgcattgcga caaactgcat 300

gttgacccgg aaaacttccg tctgctgggt aacgttctgg tttgcgttct ggctcatcat 360

ttcggtaaag agtttactcc gccggttcag gctgcttacc agaaagttgt tgctggtgtt 420

gctaacgctc tggctcataa ataccattaa taggaattc 459

<210> 15
<211> 21
<212> DNA
<213> Artificial

<220>
<223> Primer

<400> 15
gactggttcc aattgacaag c 21

<210> 16
<211> 30
<212> DNA
<213> Artificial

<220>
<223> Primer

<400> 16
cggttaactt caaactgctg tctcattgcc 30

<210> 17
<211> 23
<212> DNA
<213> Artificial

<220>
<223>  Primer

<400>  17
gccaggaatt tgtccagaga agc                                               23


<210>  18
<211>  23
<212>  DNA
<213>  Artificial

<220>
<223>  Primer

<400>  18
ccgtctgctg ggtaacgttc tgg                                               23


<210>  19
<211>  24
<212>  DNA
<213>  Artificial

<220>
<223>  Primer

<400>  19
gcaacaccag caacaacttt ctgg                                              24